# EUROPÄISCHE PATENTSCHRIFT

(11) **EP 2 416 755 B1**
(45) Veröffentlichungstag und Bekanntmachung des Hinweises auf die Patenterteilung: **27.03.2013**
(21) Anmeldenummer: 10711850.7
(22) Anmeldetag: 31.03.2010
(51) Int. Cl.: A61K 9/00, A61K 9/20, A61K 31/519

(54) **SCHMELZTABLETTE, ENTHALTEND EIN SILDENAFIL-SALZ**
MELTING TABLET CONTAINING A SILDENAFIL SALT
COMPRIME ORODISPERSIBLE CONTENANT UN SEL DE SILDENAFIL

(30) Priorität: 06.04.2009 DE 102009016584
(43) Veröffentlichungstag der Anmeldung: 15.02.2012
(73) Patentinhaber: Ratiopharm GmbH, 89079 Ulm (DE)
(72) Erfinder: WINTER, Sven, 59269 Neubeckum (DE); SCHEIWE, Max-Werner, Dr., 79689 MauIburg (DE)
(74) Vertreter: Aechter, Bernd
(86) Internationale Anmeldenummer: PCT/EP2010/002081
(87) Internationale Veröffentlichungsnummer: WO 2010/115576

(56) Entgegenhaltungen:
- EP-A1- 1 120 120
- WO-A1-00/54777
- WO-A1-98/30209
- WO-A1-03/072084
- WO-A1-2004/017976
- WO-A1-2007/002125
- WO-A2-2007/057762
- US-A1- 2006 127 479

## Beschreibung

Die Erfindung betrifft eine Schmelztablette, enthaltend die Komponenten pharmazeutisch verträgliches Sildenafil-Salz (a), ein oder mehrere polymere Adsorptionsmittel (b), insbesondere Kationenaustauscherharz, ein oder mehrere Süßungsmittel (c), und ein oder mehrere Aromamittel (d) sowie bevorzugt ein oder mehrere Schleimstoffe (e) und ein oder mehrere Gleitmittel (f). Ferner betrifft die Erfindung ein Verfahren zur Herstellung von Schmelztabletten enthaltend ein pharmazeutisch verträgliches Sildenafil-Salz.

Der IUPAC-Name von Sildenafil [INN] ist 1-{[3-(1-Methyl-7-oxo-3-propyl-6,7-dihydro-1H-pyrazolo-[4,3-d]pyrimidin-5-yl)-4-ethoxyphenyl]-sulfonyl}-4-methylpiperazin. Die chemi-sche Struktur von Sildenafil wird in nachstehender Formel (1) dargestellt:

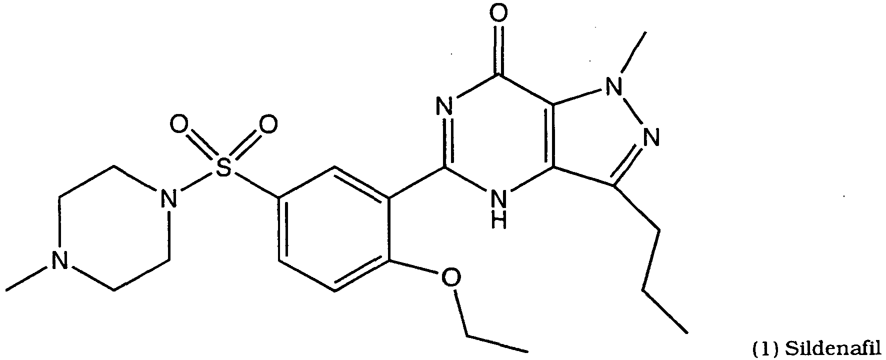

Sildenafil ist ein potenter selektiver Hemmer der cGMP-spezifischen Phosphodiesterase vom Typ 5 (PDE-5), die für die Herabsetzung von cGMP im Corpus Cavernosum verantwortlich ist. Sildenafil wird unter dem Handelsnamen Viagra^{®} zur Behandlung der erektilen Dysfunktion vertrieben.

Im Stand der Technik werden verschiedene Formulierungen für PDE-5 Inhibitoren, insbesondere für Sildenafil vorgeschlagen. WO 2007/02125 beschreibt Sublingualtabletten, die PDE-5 Inhibitoren, wie beispielsweise Sildenafil-Citrat, enthalten. Die in den Beispielen aufgeführten hergestellten Tabletten weisen jedoch einen für den Patienten nicht akzeptablen, bitteren Geschmack auf.

Zur Maskierung des bitteren Geschmacks schlägt WO 98/030209 eine orale Darreichungsform vor, die einen wirkstoffhaltigen Kern, eine innere Überzugsschicht und eine äußere Überzugsschicht umfasst, wobei die äußere Überzugsschicht ein speichelunlösliches Polymer enthält. Die Herstellung der vorgeschlagenen oralen Darreichungsform erfordert jedoch einen unerwünscht hohen technischen Aufwand.

WO 03/072084 A1 und WO 2004/017976 A1 beschreiben ein Verfahren zu Herstellung von rasch zerfallenden Tabletten, wobei zunächst Sildenafilgranulate mittels Feuchtgranulierung hergestellt werden und diese dann mit bereits vorgranuliertem Sprengmittel vermischt werden. Das offenbarte Verfahren ist technische aufwendig und ermöglicht zudem nur einen beschränkten Wirkstoffgehalt. Eine Geschmacksmaskierung des Wirkstoffs wird über die Zugabe eines Gegenions zur Löslichkeitsverminderung und durch Zugabe eines pH-erhöhenden Zusatzes (Na-Carbonat) erzielt.

Die im Stand der Technik vorgeschlagenen Möglichkeiten zur Geschmacksmaskierung von löslichen Sildenafil-Salzen sind jedoch gemäß EP 0 960 621 A1 allesamt unbefriedigend. Demgemäß wird in EP 0 960 621 A1 und EP 1 120 120 A1 die Verwendung des Wirkstoffs Sildenafil als freie Base vorgeschlagen. Durch die Unlöslichkeit der freien Base ist Sildenafil dann geschmacklos und die Notwendigkeit der unzureichenden Bittermaskierung wird dadurch überwunden. Die Verwendung von wasserunlöslichen Wirkstoffen ist jedoch häufig unerwünscht, beispielsweise im Hinblick auf ein schnelles Anflutverhalten.

Die Dokumente EP 1 120 120 A1 (Eisai Co. Ltd.), WO 2007/002125A1 (Schering Corp.) WO 98/30209 A1 (Pfizer Pharma (JP)), WO 2004/017976 A1 (Phoqus Pharmaceuticals Ltd (GB)) und WO 03/072084 A1 (Phoqus Ltd (GB)) beschreiben sich schnell auflösende pharmazeutische Darrei- chungsformen eines Wirkstoffes, wie zum Beispiel Sildenafil. Diese Darreichungsformen können unter anderem oral verabreicht werden.

Die Dokumente WO 00/54777 A1 (Pentech Pharmaceuticals Inc.) und WO 2007/057762 A2 (Pfizer Ltd (GB)) beschreiben Sildenafil-haltige Zusammensetzungen mit kontrollierter Freigabe des Wirkstoffes nach beispielsweise einer sublingualen oder bukkalen Aufnahme.

Dokument US 2006/127479 A1 (Kumaraperumal Natrajan et al.) i befasst sich mit einer pharmazeutischen Zusammensetzung, bei welcher der bittere Geschmack eines Wirkstoffs, wie Cetirizin, maskiert wird.

Zusammenfassend ist festzustellen, dass die im Stand der Technik vorgeschlagenen Formulierungen Nachteile aufweisen. Aufgabe der Erfindung war es daher, diese Nachteile zu überwinden.

Im Speziellen war es eine Aufgabe der vorliegenden Erfindung eine orale Darreichungsform mit kurzer Zerfallszeit bereit zu stellen. Die orale Darreichungsform soll mit hohem Wirkstoffgehalt herstellbar sein. Die Verwendung der freien Sildenafil-Base sollte vermieden werden.

Ferner war es eine Aufgabe der Erfindung, eine orale Darreichungsform bereit zu stellen, die einen akzeptablen Geschmack beim Patienten erzielt (d.h. der Patient sollte keinen bitteren Geschmack empfinden).

Der akzeptable Geschmack soll auch mit einer zuckerfreien Formulierung ermöglicht werden.

Schließlich sollen die vorstehend genannten Aufgaben mittels eines technisch einfachen (und somit kostengünstigen) Verfahrens in hoher Raum-Zeit-Ausbeute gelöst werden. Die Aufgaben konnten durch Herstellung einer Schmelztablette enthaltend ein pharmazeutisch verträgliches Salz von Sildenafil sowie weitere pharmazeutische Hilfsstoffe, insbesondere mittels Direktverpressung, gelöst werden.

Gegenstand der Erfindung ist daher eine Schmelztablette, enthaltend die Komponenten
(a) pharmazeutisch verträgliches Sildenafil-Salz;
(b) Ionenaustauscherharz als polymeres Adsorptionsmittel;
(c) Süßungsmittel; und
(d) Aromamittel;
sowie gegebenenfalls Schleimstoff (e) und/oder Gleitmittel (f).

Ferner ist Gegenstand der Erfindung ein Verfahren zur Herstellung einer Schmelztablette enthaltend ein pharmazeutisch verträgliches Salz von Sildenafil, umfassend die Schritte:
(i) Vermischen eines pharmazeutisch verträglichen Sildenafil-Salzes mit pharmazeutischen Hilfsstoffen, und
(ii) Verpressen der Mischung,
wobei das Verfahren in Abwesenheit von Lösungsmitteln ausgeführt wird.

Wie vorstehend erläutert, betrifft die vorliegende Erfindung eine Schmelztablette. Unter dem Begriff "Schmelztablette" wird im Rahmen dieser Erfindung eine Schmelztablette verstanden wie im Europäischen Arzneibuch, 6. Auflage, Grundwerk 2008 definiert. Somit handelt es sich bei einer Schmelztablette um eine orale, in der Mundhöhle zerfallende Tablette. Bei der Schmelztablette handelt es sich um eine nicht überzogene Tablette, das heißt nicht beschichtet oder unbefilmt.

Gemäß Ph. Eur. 6. Ausgabe müssen Schmelztabletten innerhalb von 3 Minuten zerfallen. Im Rahmen dieser Erfindung ist es bevorzugt, dass die erfindungsgemäßen Schmelz-tabletten eine Zerfallszeit von weniger als 30 Sekunden, insbesondere von weniger als 20 Sekunden im Mund aufweisen.

Die Zerfallszeit wird gemäß Ph. Eur. 6. Auflage Kapitel 2.9.1 Prüfung A bestimmt. Sofern im Rahmen dieser Anmeldung auf die durchschnittliche Zerfallszeit genommen wird, ist darunter der Mittelwert aus der Zerfallszeitbestimmung von 10 Tabletten zu verstehen.

Die erfindungsgemäßen Schmelztabletten weisen üblicherweise ein Gesamtgewicht von weniger als 1000 mg auf, mehr bevorzugt von weniger als 750 mg, insbesondere von weniger als 500 mg. Üblicherweise weisen die erfindungsgemäßen Schmelztabletten ein Gewicht von mehr als 50 mg, bevorzugt 100 mg oder mehr, insbesondere mehr als 150 mg auf.

Das Gewichtsverhältnis von Wirkstoff zu Hilfsstoffen beträgt in den erfindungsgemäßen Schmelztabletten üblicherweise 4 : 1 bis 1 : 5, bevorzugt von 2 : 1 bis 1 : 3, insbesondere 1 : 1 bis 1 : 2.

Die erfindungsgemäßen Schmelztabletten sind daher eindeutig von so genannten "Kautabletten" zu unterscheiden, da diese üblicherweise ein höheres Gewicht (ca. 1,5 bis 3 g) und eine längere Zerfallszeit aufweisen.

Die erfindungsgemäßen Schmelztabletten weisen üblicherweise eine Härte von 25 bis 80 N, bevorzugt von 35 bis 70 N, mehr bevorzugt von 40 bis 65 N, insbesondere von 45 bis 60 N auf. Die Härte wird üblicherweise gemäß Ph. Eur. 6. Auflage Kapitel 2.9.8 bestimmt. Sofern im Rahmen dieser Anmeldung auf die mittlere Härte Bezug genommen wird, ist darunter der Mittelwert aus der Härtebestimmung von 10 Tabletten zu verstehen.

Zudem zeigen die resultierenden Tabletten bevorzugt eine Friabilität von kleiner 5 %, besonders bevorzugt von kleiner 3 %, insbesondere kleiner 1 %, auf. Die Friabilität wird gemäß Ph.Eur. 6.0, Abschnitt 2.9.7 bestimmt.

Die erfindungsgemäße Schmelztablette enthält als Komponente (a) ein pharmazeutisch verträgliches Sildenafil-Salz.

Beispiele für pharmazeutisch verträgliche Salze sind Ammoniumsalze, Hydrochloride, Carbonate, Hydrogencarbonate, Acetate, Lactate, Butyrate. Propionate, Sulfate, Methansulfonate, Citrate, Tartrate, Nitrate, Sulfonate, Oxalate und/oder Succinate.

Bevorzugt verwendet wird Sildenafil-Citrat und Sildenafil-Hydrochlorid. Insbesondere wird Sildenafil-Citrat verwendet. Zudem können Solvate oder Hydrate der vorstehend genannten Salze als Komponente (a) verwendet werden.

Die erfindungsgemäßen Schmelztabletten enthalten Komponente (a) üblicherweise in einem Anteil von 20 bis 70 Gew.-%, bevorzugt 25 bis 60 Gew.-%, insbesondere 28 bis 55 Gew.-%, bezogen auf das Gesamtgewicht der Schmelztablette. Ferner enthalten die erfindungsgemäßen Schmelztabletten Komponente (a) üblicherweise in einer Menge von 25 bis 250 mg, mehr bevorzugt von 50 bis 150 mg. Insbesondere enthalten die erfindungsgemäßen Schmelztabletten 25 mg, 50 mg oder 100 mg an Komponente (a). bezogen auf den Gehalt an Sildenafil-Base. Beispielsweise entspricht 35.12 mg Sildenafil-Citrat umgerechnet 25 mg Sildenafil-Base.

Die erfindungsgemäßen Schmelztabletten enthalten als Komponente (b) ein polymeres Adsorptionsmittel. Im Allgemeinen ist darunter ein polymerer Stoff (d.h. Stoff mit mehr als zwei sich wiederholenden Monomereinheiten) zu verstehen, der in der Lage ist, an die Komponente (a) zu adsorbieren.

Bevorzugt handelt es sich bei dem polymeren Adsorptionsmittel um ein hydrophiles Polymer.

Darunter sind Polymere zu verstehen, die hydrophile Gruppen aufweisen. Beispiele für geeignete hydrophile Gruppen sind Hydroxy, Amino, Carbonsäure oder Carboxylat (nachfolgend auch als Carboxyl/Carboxylat ausgedrückt), Sulfonsäure oder Sulfonat (nachfolgend auch als Sulfonsäure/Sulfonat ausgedrückt). Ferner weist das Polymer (b) bevorzugt ein zahlenmittleres Molekulargewicht von 10³ bis 10²¹ g/mol, mehr bevorzugt von 10⁶ bis 10¹⁸ g/mol, auf. Das Polymer (b) kann linear oder bevorzugt quervernetzt sein. Das Polymer (b) weist in letzterem Fall bevorzugt einen Quervernetzungsgrad von 0,01 bis 10 %, insbesondere von 0,1 bis 5 %, auf. (Quervernetzungsgrad = Anzahl an Kohlenstoffatomen, die mehr als an eine Kette anknüpfen / Anzahl an Kohlenstoffatomen in der Polymerkette gesamt).

Die erfindungsgemäßen Schmelztabletten enthalten als Komponente (b) ein Ionenaustauscherharz. Ein Ionenaustauscherharz ist ein Polymer, mit dem gelöste Ionen gegen andere Ionen gleicher Ladungsart ersetzt werden können.

Mehr bevorzugt wird als Komponente (b) ein Kationenaustauscherharz verwendet. Unter Kationenaustauscherharz ist ein Polymer zu verstehen, das funktionelle Gruppen mit einem abdissoziierbaren Kation enthält. Beispiele für diese funktionellen Gruppen sind Sulfonsäuregruppen/Sulfonatgruppen oder Carboxylgruppen/Carboxylatgruppen. Somit wird als Komponenten (b) bevorzugt ein Polymer verwendet, das Carboxyl-gruppen/Carboxylatgruppen und/oder Sulfonylgruppen/Sulfonatgruppen enthält. Sofern Carboxylat oder Sulfonatgruppen vorliegen, können z.B. Ammonium-, Alkali- und Erdalkaliionen als Gegenionen dienen, bevorzugt sind Natrium und Kalium, insbesondere Kalium. Natriumstärkeglykolat ist kein polymeres Adsorptionsmittel (b) im Sinne dieser Erfindung.

In einer besonders bevorzugten Ausführungsform handelt es sich bei dem polymeren Adsorptionsmittel (b) um ein Copolymer, erhältlich durch Copolymerisation von Methacrylsäure und Divinylbenzol. Ein derartiges Copolymer ist unter der Bezeichnung Polacrilin bekannt. Insbesondere wird im Rahmen dieser Erfindung Polacrilin in Form des Kaliumsalzes (Polacrilin-Kalium, insbesondere gemäß US Pharmacopeia monographiert) verwendet.

Polacrilin-Kalium kann durch folgende Strukturformel veranschaulicht werden. 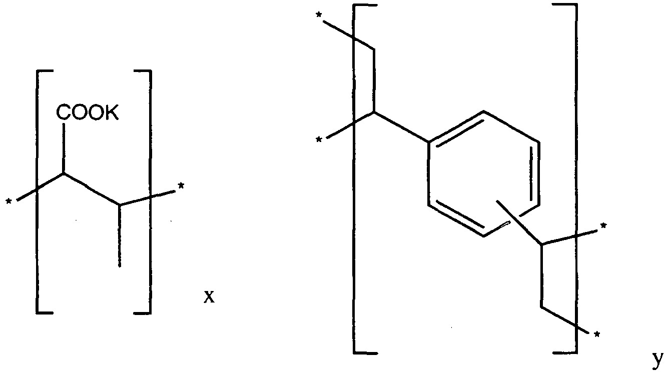 wobei x und y natürliche Zahlen sind, beispielsweise von 10¹ bis 10²⁰, bevorzugt von 10⁶ bis 10⁸. Das Verhältnis von x zu y beträgt üblicherweise 50 : 1 bis 1 : 1, bevorzugt 20 : 1 bis 2 : 1, besonders bevorzugt 10 : 1 bis 3 : 1.

Üblicherweise wird das polymere Adsorptionsmittel (b) in der erfindungsgemäßen Schmelztablette in einer Menge (b) von 1 bis 60 Gew.-%, bevorzugt von 5 bis 50 Gew.-%, mehr bevorzugt von 8 bis 45 Gew.-%, besonders bevorzugt von 10 bis 40 Gew.-% und insbesondere von 15 bis 35 Gew.-%, bezogen auf das Gesamtgewicht der Schmelztablette, verwendet.

Die erfindungsgemäßen Schmelztabletten enthalten als Komponente (c) ein oder mehrere Süßungsmittel. Als Süßungsmittel werden im Allgemeinen Stoffe bezeichnet, die beim Patienten üblicherweise einen süßen Geschmackseindruck bei Einnahme bewirken.

Bevorzugt werden Süßungsmittel verwendet, die eine Süßkraft von 0,2 bis 13000, bevorzugt von >1 bis 4000, insbesondere von 10 bis 3000 haben, bezogen auf die Süßkraft des Rohrzuckers (= 1,0).

Beispiele sind Milchzucker (0,27-0,3), Glycerin (0,5-0,8), D-Glucose (0,5-0,6), Maltose (0,6), Galactose (0,6), Invertzucker (0,8-0,9), Rohrzucker (1,0), Xylit (1,0), D-Fructose (1,0-1,5), Natriumcyclamat (30), D-Tryptophan (35), Chloroform (40), Glycyrrhizin (50), Acesulfam (130), Aspartam (180-200), Dulcin (200), Suosan^{®} (350), Saccharin

(Natriumsalz) (400-500), Saccharin (Ammoniumsalz) (600), 1-Brom-5-Nitroanilin (700), Naringindedehydrochalcon (1000-1500). Thaumatin, Monellin (Peptide) (3000), P-4000, n-Propoxy-2-Amino-4-Nitrobenzol (4000), Alitam (3000) und/oder Neotam (13.000). Der Zahlenwert in Klammern gibt die Süßkraft bezogen auf Rohzucker an. Ebenfalls können Thaumatin und/oder Neohesperidin DC verwendet werden.

In der erfindungsgemäßen Schmelztablette wird die Komponente (c) üblicherweise in einer Menge von 0,01 bis 10 Gew.-%, mehr bevorzugt von 0,1 bis 5 Gew.-%, insbesondere von 0,5 bis 2 Gew.-%, bezogen auf das Gesamtgewicht der Tablette, verwendet.

In einer bevorzugten Ausführungsform enthält die erfindungsgemäße Schmelztablette mehrere verschiedene Süßungsmittel, besonders bevorzugt enthält die erfindungsgemäße Tablette 2, 3 oder 4 verschiedene Süßungsmittel.

In einer mehr bevorzugten Ausführungsform enthält die Komponenten (c)
(c-1) ein Süßungsmittel mit sofortiger Süße und
(c-2) ein Süßungsmittel mit verzögerter Süße.

Die Komponenten (c-1) und (c-2) können üblicherweise im Gewichtsverhältnis von 5:1 bis 1:5, bevorzugt von 3:1 bis 1:3, eingesetzt werden.

Beispiele für ein Süßungsmittel mit sofortiger Süße (c-1) sind Saccharin-Natriumsalz, Saccharin-Ammoniumsalz, Sucralose, Neotam, Alitam, Aspartam, Cyclamat. Thaumatin und/oder Acesulfam.

Beispiele für Süßungsmittel mit verzögerter Süße (c-2) sind Glycyrrhizin oder Derivate davon, insbesondere Glycyrrhizin in Form des Mono-Ammoniumsalzes, Thaumatin und Neohesperidin DC.

Die erfindungsgemäßen Schmelztabletten enthalten als Komponente (d) ein oder mehrere Aromamittel. Im Rahmen dieser Anmeldung ist der Begriff "Aromamittel" wie in der Richtlinie des Rates 88/388/EWG "Aromastoffe" vom 22. Juni 1988 definiert zu verstehen.

Aromamittel (d) können wie folgt gewonnen werden:
i) durch geeignete physikalische Verfahren (einschließlich Destillation und Extraktion mit Lösungsmitteln) oder enzymatische bzw. mikrobiologische Verfahren aus Stoffen pflanzlichen oder tierischen Ursprungs, die als solche verwendet oder mittels herkömmlicher Lebensmittelzubereitungsverfahren (einschließlich Trocknen, Rösten und Fermentierung) für den menschlichen Verzehr verarbeitet werden;
ii) durch chemische Synthese oder durch Isolierung mit chemischen Verfahren, wobei ihre chemische Beschaffenheit mit einer Substanz identisch ist, die in einem Stoff pflanzlichen oder tierischen Ursprungs im Sinne von Ziffer i) natürlich vorkommt;
iii) durch chemische Synthese, wobei jedoch ihre chemische Beschaffenheit nicht mit einer Substanz identisch ist, die in einem Stoff pflanzlichen oder tierischen Ursprungs im Sinne von Ziffer i) natürlich vorkommt."

Ein Aromamittel besteht kann hierbei aus einem oder bevorzugt aus mehreren chemischen Verbindungen bestehen. Beispielsweise kann Pfefferminzaroma ein Kollektiv aus mehr als 10 chemischen Verbindungen enthalten.

In der erfindungsgemäßen Schmelztablette wird die Komponente (d) üblicherweise in einer Menge von 0,001 bis 5 Gew.-%, mehr bevorzugt von 0,1 bis 4 Gew.-%, insbesondere von 0,2 bis 2 Gew.-%, bezogen auf das Gesamtgewicht der Tablette verwendet.

In einer bevorzugten Ausführungsform enthält die erfindungsgemäße Schmelztablette mehrere verschiedene Aromamittel (d.h. mehrere verschiedene Aromarichtungen), besonders bevorzugt enthält die erfindungsgemäße Tablette 2, 3 oder 4 verschiedene Aromamittel.

Bevorzugte Kombinationen von Aromamitteln sind z.B. Aroma Pfefferminz mit Aroma Menthol, Aroma Pfefferminz mit Aroma Zitrone, Aroma Pfefferminz mit Aroma Menthol und Aroma Zitrone, Aroma Gartenminze mit Aroma Zitrone, Aroma Gartenminze mit Aroma Menthol, Aroma Gartenminze mit Aroma Zitrone und Aroma Menthol, Aroma Grapefruit mit Aroma Pfefferminze, Aroma Grapefruit mit Aroma Menthol und Aroma Pfefferminze, Aroma Grapefruit mit Aroma Gartenminze, Aroma Grapefruit mit Aroma Gartenminze und Aroma Menthol.

In einer bevorzugten Ausführungsform enthält die erfindungsgemäße Schmelztablette ferner ein oder mehrere Schleimstoffe (als Komponente (e)). Unter Schleimstoffe werden im Rahmen dieser Erfindung Stoffe verstanden, die durch Erhöhung der Viskosität oder durch Einhüllung den Kontakt von Arzneistoffen mit den Zungenpapillen verringern. Dadurch wird üblicherweise eine Verringerung der Intensität der Geschmacksempfindung erzielt.

In einer möglichen Ausführungsform werden als Komponente (e) Stoffe gewählt, die als 2 Gew.-%ige Lösung oder Mischung in destilliertem Wasser zu einer Viskosität von mehr als 2 mPa/s, bevorzugt von mehr als 4 mPa/s, insbesondere von mehr als 6 mPa/s führen, gemessen bei 25 °C, gemäß Ph. Eur. 6. Auflage Kapitel 2.2.10 bestimmt.

Als Schleimstoffe (e) können bevorzugt natürliche Gummis, Cellulosenderivate, Alginate und/oder nichtionische Hydrokolloide verwendet werden.

Beispiele für Schleimstoffe (e) sind Agar, Alginsäure, Alginat, Chicle, Carageenan, Dammar, Eibisch Extrakte, Gellan (E 418), Guarkernmehl (E 412), Gummi Arabicum (E 414). Gummi aus Wegerichkernspelze, Gummi aus Fichtensaft, Johannisbrotkernmehl E 410), Karaya (E 416), Konjakmehl (E 425), aus der Konjakwurzel gewonnen, Tarakernmehl (E 417), Traganth (E 413), Xanthan Gummi (E 415), bevorzugt hergestellt durch bakterielle Fermentation, Guar Gummi und/oder Lecithin.

Als Schleimstoffe auf Basis von Cellulosederivate werden z.B. Carboxymethylcellulose, Hydroxyethylcellulose und/oder Methylcellulose verwendet.

Besonders bevorzugt wird Gummi Arabicum als Komponente (e) verwendet.

In der erfindungsgemäßen Schmelztablette wird die Komponente (e) üblicherweise in einer Menge von 0 bis 15 Gew.-%, mehr bevorzugt von 0,1 bis 10 Gew.-%, insbesondere von 0,5 bis 5 Gew.-% verwendet, bezogen auf das Gesamtgewicht der Tablette.

In einer möglichen Ausführungsform enthält die erfindungsgemäße Schmelztablette mehrere verschiedene Schleimstoffe, besonders bevorzugt enthält die erfindungsgemäße Tablette 2 oder 3 verschiedene Schleimstoffe.

In einer bevorzugten Ausführungsform enthält die erfindungsgemäße Schmelztablette ferner ein Gleitmittel (als Komponente (f)). Gleitmittel dienen hierbei als Mittel zur Verbesserung der Pulverfließfähigkeit (=Fließreguliermittel) und/oder als Schmiermittel.

Fließreguliermittel (f) haben die Aufgabe, in einem Tablettiergemisch sowohl die interpartikuläre Reibung (Kohäsion) zwischen den einzelnen Partikeln als auch das Haften dieser an den Wandflächen der Pressform (Adhäsion) zu vermindern. Ein Beispiel für einen Zusatz zur Verbesserung der Pulverfließfähigkeit ist disperses Siliciumdioxid. Bevorzugt wird Siliciumdioxid mit einer spezifischen Oberfläche von 50 bis 400 m²/g bestimmt nach Gasadsorption gemäß Ph. Eur., 6. Auflage 2.9.26. verwendet. In diesem Zusammenhang wurde unerwartet gefunden, dass durch die Verwendung von Siliciumdioxid mit einer spezifischen Oberfläche von 50 bis 400 m²/g die Menge an Füllstoff vorteilhaft reduziert werden kann.

Ferner können als Komponente (f) Schmiermittel verwendet werden. Schmiermittel dienen im Allgemeinen zur Verringerung der Gleitreibung. Insbesondere soll die

Gleitreibung vermindert werden, die beim Tablettieren einerseits zwischen den sich in der Matrizenbohrung auf und ab bewegenden Stempeln und der Matrizenwand sowie andererseits zwischen Tablettensteg und Matrizenwand besteht. Geeignete Schmiermittel stellen z.B. Stearinsäure, Adipinsäure, Natriumstearylfumarat, Magnesiumstearat und/oder Calciumstearat dar.

In der erfindungsgemäßen Schmelztablette wird die Komponente (f) üblicherweise in einer Menge von 0 bis 10 Gew.-%, mehr bevorzugt von 0,1 bis 5 Gew.-%, insbesondere von 1,0 bis 3 Gew.-% verwendet, bezogen auf das Gesamtgewicht der Tablette.

Die erfindungsgemäße Schmelztablette kann aus den Komponenten (a) bis (d) sowie gegebenenfalls (e) und (f) bestehen. Gegebenenfalls kann die erfindungsgemäße Tablette jedoch weitere übliche, pharmazeutische Hilfstoffe enthalten.

Beispiele hierfür sind Sprengmittel, Füllstoffe und Säuerungsmittel.

Als Sprengmittel werden im Allgemeinen Stoffe bezeichnet, die den Zerfall einer Darreichungsform, insbesondere einer Tablette, nach Einbringen in Wasser beschleunigen. Geeignete Sprengmittel sind z.B. organische Sprengmittel wie mikrokristalline Cellulose, Stärke, vorverkleisterte Stärke, Natriumcarboxymethylstärke, Natriumcarboxymethylcellulose und Crospovidone. Bevorzugt wird mikrokristalline Cellulose als Sprengmittel verwendet. In einer möglichen Ausführungsform ist es bevorzugt, dass auf so genannte "Supersprengmittel", insbesondere auf Croscarmellose und Crospovidone, verzichtet wird.

Die erfindungsgemäßen Schmelztabletten enthalten Sprengmittel, insbesondere Supersprengmittel (z.B. Croscarmellose und Crospovidone) üblicherweise in einer Menge von 0 bis 25 Gew.-%, mehr bevorzugt von 1 bis 15 Gew.-%, insbesondere von 2 bis 10 Gew.-%, bezogen auf das Gesamtgewicht der Tablette. Alternativ enthalten die erfindungsgemäßen Schmelztabletten im Falle von mikrokristalliner Cellulose als Sprengmittel diese in einer Menge von 0 bis 90 Gew.-%, mehr bevorzugt von 10 bis 75 Gew.-%, insbesondere von 20 bis 60 Gew.-%.

Unter Füllstoffe sind im Allgemeinen Stoffe zu verstehen, die zur Bildung des Tablettenkörpers bei Tabletten mit geringen Wirkstoffmengen (z.B. kleiner 70 Gew.-%) dienen. Das heißt, Füllstoffe erzeugen durch "Strecken" der Wirkstoffe eine ausreichende Tablettiermasse. Füllstoffe dienen üblicherweise also dazu, eine geeignete Tablettengröße zu erhalten.

Beispiele für bevorzugte Füllstoffe sind Lactose, Stärke, Stärkederivate, Calciumphosphat. Saccharose, Calciumcarbonat, Calciumsilikat, Magnesiumcarbonat, Magnesiumoxid, Maltodextrin, Calciumsulfat, Dextrate, Dextrin, Dextrose, hydrogeniertes Pflanzenöl, Kaolin und/oder bevorzugt Zuckeralkohole. Beispiele für geeignete Zuckeralkohole sind Mannitol, Sorbitol, Xylitol, Isomalt, Glucose, Fructose, Maltose und Gemische daraus. Besonders bevorzugt wird mikrokristalline Cellulose (sofern nicht bereits als Sprengmittel verwendet) und Calciumsilikat als Füllstoff verwendet.

Die erfindungsgemäßen Schmelztabletten enthalten Füllstoffe üblicherweise in einer Menge von 0 bis 30 Gew.-%, mehr bevorzugt von 1 bis 20 Gew.-%, insbesondere von 2 bis 10 Gew.-%, bezogen auf das Gesamtgewicht der Tablette.

Säuremittel dienen zur Erzielung einer sauren Geschmackskomponente. Beispiele für Säuremittel sind Citronensäure, Weinsäure und Salze davon.

Die erfindungsgemäßen Schmelztabletten enthalten Säuremittel üblicherweise in einer Menge von 0 bis 15 Gew.-%, mehr bevorzugt von 1 bis 10 Gew.-%, insbesondere von 2 bis 5 Gew.-%, bezogen auf das Gesamtgewicht der Tablette.

Es liegt in der Natur von pharmazeutischen Hilfsstoffen, dass diese teilweise mehrere Funktionen in einer pharmazeutischen Formulierung wahrnehmen. Im Rahmen dieser Erfindung gilt zur unzweideutigen Abgrenzung daher bevorzugt die Fiktion, dass ein Stoff, der als ein bestimmter Hilfsstoff verwendet wird, nicht zeitgleich auch als weiterer pharmazeutischer Hilfsstoff eingesetzt wird. Beispielsweise wird Mannitol - sofern als Füllstoff eingesetzt, nicht auch als Süßungsmittel verwendet. Ebenfalls wird Polacrilin - sofern als polymeres Adsorptionsmittel (b) eingesetzt - nicht auch zusätzlich als Sprengmittel eingesetzt (obwohl Polacrilin auch eine gewisse Sprengwirkung zeigt).

In einer bevorzugten Ausführungsform werden in der erfindungsgemäßen Schmelztablette die Hilfsstoffe so gewählt, dass die erfindungsgemäße Schmelztablette frei von Sacchariden ist.

Gegenstand der Erfindung ist nicht nur die erfindungsgemäße Schmelztablette sondern auch ein Verfahren zu deren Herstellung. Besonders bevorzugt wird die erfindungsgemäße Tablette mittels Direktverpressung hergestellt.

Gegenstand der Erfindung ist daher ein Verfahren zur Herstellung einer Schmelztablette enthaltend ein pharmazeutisch verträgliches Salz von Sildenafil, umfassend die Schritte:
(i) Vermischen eines pharmazeutisch verträglichen Sildenafil-Salzes mit pharmazeutischen Hilfsstoffen, und
(ii) Verpressen der Mischung,
wobei das Verfahren in Abwesenheit von Lösungsmitteln ausgeführt wird.

Bevorzugt werden im Schritt (i) des erfindungsgemäßen Verfahrens die Komponenten
(a) pharmazeutisch verträgliches Sildenafil-Salz;
(b) polymeres Adsorptionsmittel, bevorzugt Kationenaustauscherharz;
(c) Süßungsmittel;
(d) Aromamittel;
(e) gegebenenfalls Schleimstoff; und
(f) gegebenenfalls Gleitmittel vermischt.

Grundsätzlich finden für das erfindungsgemäße Verfahren alle vorstehenden Erläuterungen zu bevorzugten Ausführungsformen der erfindungsgemäßen Tablette Anwendung, beispielsweise betreffend Art und Menge der Komponenten (a) bis (f) und der möglichen Zugabe von weiteren Hilfsstoffen wie Sprengmitteln, Füllstoffen und Säuremitteln.

Im Schritt (i) des erfindungsgemäßen Verfahrens werden Sildenafil-Salz (a) und pharmazeutische Hilfsstoffe vermischt. Die Vermischung kann in üblichen Mischern erfolgen. Beispielsweise kann die Vermischung in Zwangsmischern oder Freifallmischern erfolgen, z.B. mittels Turbula^{®} T 10B (Bachofen AG, Schweiz).

In einer bevorzugten Ausführungsform des erfindungsgemäßen Verfahrens werden vor der Durchführung des Schrittes (i) die Komponenten (a) und (b) miteinander vermischt. Diese Vorvermischung erfolgt beispielsweise in Zwangsmischern oder in Freifallmischern. Bei Vermischung in Zwangsmischern werden üblicherweise Zeiten von 3 bis 15 Minuten benötigt, um eine homogene Mischung zu erzielen. Bei Verwendung von Freifallmischern z.B. mittels Turbula^{®} T 10B (Bachofen AG, Schweiz) oder Containermischern z.B. mittels CM 500 (J. Engelsmann AG, Deutschland) oder Fassmischern z.B. Typ Rhönradmischer (J. Engelsmann AG, Deutschland) werden üblicherweise Zeiten von 10 bis 20 Minuten benötigt, um die homogene Mischung zu erzeugen.

Die Mischbedingungen der "Vorvermischung" (der Komponenten (a) und (b)) werden üblicherweise so gewählt, dass mindestens 50 % der Anzahl des eingesetzten Sildenafil-Salzes Teilchen an das polymere Adsorptionsmittel (bevorzugt cohäsiv) gebunden sind, mehr bevorzugt mindestens 80 % der Teilchen, besonders bevorzugt mindestens 95 % der Teilchen, insbesondere mindestens 99 % der Teilchen.

In Schritt (ii) erfolgt ein Verpressen der Mischung (aus Schritt (i)), d.h. eine Kompression zu Tabletten. Die Kompression kann mit im Stand der Technik bekannten Tablettiermaschinen erfolgen. Die Kompression erfolgt in Abwesenheit von Lösungsmitteln.

Beispiele für geeignete Tablettiermaschinen sind Exzenterpressen oder Rundlaufpressen. Beispielsweise kann eine Fette 102i (Fette GmbH, Deutschland) verwendet werden. Im Falle von Rundlaufpressen wird üblicherweise eine Presskraft von 3 bis 50 kN, bevorzugt von 7,5 bis 45 kN, angewandt.

Gegebenenfalls kann die Mischung aus Schritt (i) vor dem Verpressen kompaktiert und granuliert werden. Die Granulation erfolgt bevorzugt als Trockengranulation. Alternativ kann auch eine Feuchtgranulation erfolgen.

Das erfindungsgemäße Verfahren gewährleistet eine technisch vorteilhafte Herstellmethode. Verglichen mit der im Stand der Technik vorgeschlagenen Nassgranulierung ist es technisch weniger aufwendig und die Raum-Zeit-Ausbeute kann erhöht werden. Neben dem erfindungsgemäßen Verfahren sind auch Schmelztabletten, erhältlich nach dem erfindungsgemäßen Verfahren, Gegenstand dieser Erfindung.

Die Erfinder haben zudem festgestellt, dass sich eine Kombination aus polymerem Adsorptionsmittel (b), insbesondere in Form eines Kationenaustauscherharzes (b) mit einem Schleimstoff (d), vorteilhaft zur Geschmacksmaskierung von Medikamenten zur Behandlung der erektilen Dysfunktion (die üblicherweise einen bitteren Geschmack haben) eignet.

Gegenstand der Erfindung ist daher ferner die Verwendung einer Kombination von polymerem Adsorptionsmittel, insbesondere Kationenaustauscherharz, und Schleimstoff zur Geschmacksmaskierung von Medikamenten zur Behandlung der erektilen Dysfunktion, insbesondere zur Geschmacksmaskierung von Sildenafil-Citrat.

Die Erfindung soll durch nachfolgende Beispiele veranschaulicht werden.

### BEISPIELE

Es wurden erfindungsgemäße Schmelztabletten mittels des erfindungsgemäßen Verfahrens hergestellt. Ebenfalls wurde ein Vergleichsbeispiel ohne Verwendung des polymeren Adsorptionsmittels durchgeführt. Die eingesetzten Formulierungen und die physikalischen Eigenschaften der erhaltenen Schmelztabletten ergeben sich aus nachfolgender Tabelle.

| | **Beispiel 1** | **Beispiel 2** | **Beispiel 3** | **Vergleichsbeispiel 4** |
|---|---|---|---|---|
| Sildenafil-Citrat | 70,24 mg | 70,24 mg | 70,24 mg | 70,24 mg |
| Polacrilin-Kalium | 70,0 mg | 70,0 mg | 70,0 mg | —— |
| Glycyrrhizin | 0,2 mg | 0,2 mg | —— | 0,2 mg |
| Saccharin-Natrium | 0,2 mg | 0,2 mg | 0,4 mg | 0,2 mg |
| Gummi Arabicum | 1.5 mg | —— | 1,5 mg | 1,5 mg |
| Pfefferminz-Aroma | 0,2 mg | 0,2 mg | 0,2 mg | 0,2 mg |
| Mannitol | —— | —— | —— | 70 mg |
| MCC | 60 mg | 61,5 mg | 60 mg | 60 mg |
| Disperses Siliciumdioxid | 2,0 mg | 2,0 mg | 2,0 mg | 2,0 mg |
| Na-Stearylfumarat | 5,0 mg | 5,0 mg | 5,0 mg | 5,0 mg |
| Mittlere Härte | > 40 N | > 40 N | > 40 N | > 40 N |
| Durchschnittliche Zerfallszeit | < 30 sek | < 30 sek | < 30 sek | < 60 sek |
| Geschmack | angenehmer Pfefferminz-geschmack, nicht bitter | akzeptabler Pfefferminz-geschmack | akzeptabler Pfefferminz-geschmack, etwas bitterer Abgang | bitterer Geschmack, nicht annehmbar |

Sildenafil-Citrat wurde zusammen mit Polacrilin 10 min im Freifallmischer (Turbula^{®}) vorgemischt und ergänzt und weitere 30 min gemischt. Nach Zusatz von Magnesiumstearat wurde nochmals 2 min nachgemischt. Die Fertigmischung wurde auf einer Rundläuferpresse mit biconvex Stempeln verpresst.

Da Geschmacksempfindungen individuell verschieden sein können wurde zur Geschmacksprüfung der vorstehend durchgeführten Beispiele 10 Versuchspersonen herangezogen und das durchschnittliche Geschmacksempfinden ermittelt. Die Degustation erfolgte 1 Stunde nach der letzten Mahlzeit. Die Versuchspersonen waren alle Nichtraucher. Der Prüfraum war von neutralem Geruch, die Temperatur betrug 20 °C. Die Beurteilung der Reizempfindungen und die Ausbildung der Prüfpersonen erfolgte gemäß DIN 10950.

## Patentansprüche

1. Schmelztablette, enthaltend die Komponenten
(a) pharmazeutisch verträgliches Sildenafil-Salz;
(b) Ionenaustauscherharz als polymeres Adsorptionsmittel;
(c) Süßungsmittel; und
(d) Aromamittel.

2. Schmelztablette gemäß Anspruch 1, zusätzlich enthaltend die Komponente (e) Schleimstoff.

3. Schmelztablette gemäß Anspruch 1 oder 2, wobei pharmazeutisch verträgliches Sildenafil-Salz (a) in einer Menge von 20 bis 70 Gew.-%, bezogen auf das Gesamtgewicht der Schmelztablette, enthalten ist.

4. Schmelztablette gemäß einem der Ansprüche 1 bis 3, wobei polymeres Adsorptionsmittel (b) in einer Menge von 10 bis 50 Gew.-%, bezogen auf das Gesamtgewicht der Schmelztablette, enthalten ist.

5. Schmelztablette gemäß einem der Ansprüche 1 bis 4, wobei das polymere Adsorptionsmittel ein Kationenaustauscherharz, insbesondere Polacrilin-Kalium, ist.

6. Schmelztablette gemäß einem der Ansprüche 1 bis 5, wobei Schleimstoff (e) in einer Menge von 0,1 bis 10 Gew.-%, bezogen auf das Gesamtgewicht der Schmelztablette, enthalten ist.

7. Schmelztablette gemäß einem der Ansprüche 1 bis 6, wobei der Schleimstoff ein natürlicher Gummi ist.

8. Schmelztablette gemäß einem der Ansprüche 1 bis 7, zusätzlich enthaltend die Komponente
(f) Gleitmittel,
wobei das Gleitmittel bevorzugt Siliciumdioxid mit einer spezifischen Oberfläche von 50 bis 400 m²/g ist.

9. Schmelztablette gemäß einem der Ansprüche 1 bis 8, wobei Komponente (a) Sildenafil-Citrat ist.

10. Verfahren zur Herstellung einer Schmelztablette enthaltend ein pharmazeutisch verträgliches Salz von Sildenafil, umfassend die Schritte
(i) Vermischen von
(a) pharmazeutisch verträglichem Sildenafil-Salz;
(b) lonenaustauschharz als polymeres Adsorptionsmittel
(c) Süßungsmittel;
(d) Aromamittel;
(e) gegebenenfalls Schleimstoff; und
(f) gegebenenfalls Gleitmittel, und
(ii) Verpressen der Mischung,
wobei das Verfahren in Abwesenheit von Lösungsmitteln ausgeführt wird.

11. Verfahren gemäß Anspruch 10, wobei vor der Durchführung des Schrittes (i) die Komponenten (a) und (b) miteinander vermischt werden.

12. Schmelztablette gemäß einem der Ansprüche 1 bis 9, wobei die Schmelztablette eine Zerfallszeit von weniger als 30 Sekunden und eine Härte von 30 bis 70 Newton aufweist.

## Claims

1. Orodispersible tablet comprising the components
(a) pharmaceutically acceptable sildenafil salt;
(b) ion exchanger resin as polymeric adsorbent;
(c) sweetener; and
(d) flavoring.

2. The orodispersible tablet according to claim 1, additionally comprising component (e) mucilage.

3. The orodispersible tablet according to claim 1 or 2, wherein pharmaceutically acceptable sildenafil salt (a) is present in an amount from 20 to 70% by weight, based on the total weight of the orodispersible tablet.

4. The orodispersible table according to any one of claims 1 to 3, wherein polymeric adsorbent (b) is present in an amount of from 10 to 50% by weight, based on the total weight of the orodispersible tablet.

5. The orodispersible tablet according to any one of claims 1 to 4, wherein the polymeric adsorbent is a cation exchanger resin, in particular polacrilin potassium.

6. The orodispersible tablet according to any one of claims 1 to 5, wherein mucilage (e) is present in an amount of from 0.1 to 10% by weight, based on the total weight of the orodispersible tablet.

7. The orodispersible tablet according to any one of claims 1 to 6, wherein the mucilage is a natural gum.

8. The orodispersible tablet according to any one of claims 1 to 7, additionally comprising the component
(f) glidant,
the glidant preferably being silicon dioxide with a specific surface of from 50 to 400 m²/g.

9. The orodispersible tablet according to any one of claims 1 to 8, wherein component
(a) is sildenafil citrate.

10. Process for the preparation of an orodispersible tablet comprising a pharmaceutically acceptable salt of sildenafil, comprising the steps
(i) mixing
a) pharmaceutically acceptable sildenafil salt;
(b) ion exchanger resin as polymeric adsorbent;
(c) sweetener;
(d) flavoring;
(e) optionally mucilage; and
(f) optionally glidant, and
(ii) compressing the mixture,
the process being carried out in the absence of solvents.

11. The process according to claim 10, wherein components (a) and (b) are mixed with each other before carrying out step (i).

12. The orodispersible tablet according to any one of claims 1 to 9, wherein the orodispersible tablet has a disintegration time of less than 30 seconds and a hardness of from 30 to 70 Newton.

## Revendications

1. Comprimé orodispersible contenant les composants
(a) un sel de sildénafil compatible du point de vue pharmaceutique ;
(b) une résine échangeuse d'ions en tant que produit polymère d'adsorption;
(c) des édulcorants ; et
(d) des arômes.

2. Comprimé orodispersible selon la revendication 1, contenant en supplément le composant (e) substance mucilagineuse.

3. Comprimé orodispersible selon la revendication 1 ou la revendication 2, le sel de sildénafil (a) compatible du point de vue pharmaceutique étant contenu dans une quantité de 20 à 70 % en poids, en rapport au poids total du comprimé orodispersible.

4. Comprimé orodispersible selon l'une quelconque des revendications 1 à 3, le produit polymère d'adsorption (b) étant contenu dans une quantité de 10 à 50 % en poids, en rapport au poids total du comprimé orodispersible.

5. Comprimé orodispersible selon l'une quelconque des revendications 1 à 4, le produit polymère d'adsorption étant une résine échangeuse de cations, notamment du polacriline potassium.

6. Comprimé orodispersible selon l'une quelconque des revendications 1 à 5, la substance mucilagineuse (e) étant contenue dans une quantité de 0,1 à 10 % en poids, en rapport au poids total du comprimé orodispersible.

7. Comprimé orodispersible selon l'une quelconque des revendications 1 à 6, la substance mucilagineuse étant un caoutchouc naturel.

8. Comprimé orodispersible selon l'une quelconque des revendications 1 à 7, contenant en supplément le composant
(f) agent lubrifiant,
l'agent lubrifiant étant de préférence du dioxyde de silicium avec une surface spécifique de 50 à 400 m²/g.

9. Comprimé orodispersible selon l'une quelconque des revendications 1 à 8, le composant (a) étant du sulfate de sildénafil.

10. Procédé de production d'un comprimé orodispersible contenant un sel de sildénafil compatible du point de vue pharmaceutique, comprenant les étapes :
(i) Mélange
(a) d'un sel de sildénafil compatible du point de vue pharmaceutique ;
(b) d'une résine échangeuse d'ions en tant que produit polymère d'adsorption ;
(c) d'édulcorants ;
(d) d'arômes ;
(e) le cas échéant, d'une substance mucilagineuse ; et
(f) le cas échéant, d'un agent lubrifiant, et
(ii) Compression du mélange,
le procédé étant réalisé en l'absence de solvants.

11. Procédé selon la revendication 10, les composants (a) et (b) étant mélangés ensemble avant la réalisation de l'étape (i).

12. Comprimé orodispersible selon l'une quelconque des revendications 1 à 9, le comprimé orodispersible faisant preuve d'un temps de désintégration de moins de 30 secondes et d'une dureté de 30 à 70 Newton.
